# EUROPEAN PATENT APPLICATION

(11) **EP 1 929 997 A1**
(43) Date of publication of application: **11.06.2008**
(21) Application number: 07121728.5
(22) Date of filing: 28.11.2007
(51) Int. Cl.: A61K 9/10, A61K 9/16, A61K 9/20, A61K 9/48, A61K 31/55

(54) **Oxcarbazepine formulations**

(30) Priority: 08.12.2006 IN MU20122006
(71) Applicant: Sun Pharmaceutical Industries LTD, Mahakali Caves Road Andheri (East) Mumbai 400 093 (IN)
(72) Inventor: MUNGRE, Ashish Prabhakar, Sun Pharmaceutical Industries Ltd, c/o Sun Pharmaceutical Ind. Ltd., Andheri (EAST), 400 093, Mumbai (IN); DHARMADHIKARI, Nitin Bhalachandra, Sun Pharmaceutical Industries Ltd, c/o Sun Pharmaceutical Ind. Ltd., Andheri (EAST), 400 093, Mumbai (IN)
(74) Representative: Atkinson, Jonathan David Mark

(57) **Abstract**

A pharmaceutical dosage form comprising a mixture of a therapeutically effective amount of oxcarbazepine having median particle size ranging from about 15 µm to about 26 µm and one or more hydrophilic polymers, said mixture being formed by subjecting a suspension comprising said oxcarbazepine and a hydrophilic polymer in a solvent, to mixing in a homogenizer, optionally removing the solvent and converting the said mixture into a dosage form.

## Description

Present invention relates to improved oxcarbazepine pharmaceutical dosage forms that provide oxcarbazepine in bioavailable form.

### BACKGROUND OF THE INVENTION

Oxcarbazepine is chemically known as 10,11-Dihydro-10-oxo-5H-dibenz [b,f]azepine-5-carboxamide. It is indicated for use as monotherapy or adjunctive therapy in the treatment of partial seizures in adults and children aged 4-16 with epilepsy. Oxcarbazepine is available commercially under the trade name Trileptal^{®} in the United States of America as 150 mg, 300 mg and 600 mg film-coated tablets and a 300mg/5mL (60mg/mL) suspension. Oxcarbazepine is practically insoluble in water and achieving adequate oral bioavailability from dosage forms containing oxcarbazepine represents the problem before the instant invention.

United States Patent 7,037,525 (hereinafter referred to as '525 Patent) provides film-coated tablets which comprise a tablet core comprising a therapeutically effective dose of oxcarbazepine, preferably in a finely ground form, having a median particle size of approximately from 2 µm to 12 µm, with a maximum residue on a 40 µm sieve of up to 5 % and further excipients that are suitable for the production of granules; and a hydrophilic permeable outer coating. The tablets prepared according to this invention provide the therapeutically effective plasma levels of oxcarbazepine due to use of the specified median particle size of 2 µm to 12 µm. One problem with these formulations is that the small particle size may lead to difficulty in manufacture and handling. An additional problem with these formulations is that the small particle size may in practice result in difficulties in formulating the formulation.

PCT publication WO2002094774 (hereinafter referred to as publication number '774) discloses a dosage form for oral administration comprising oxcarbazepine and a wetting agent selected from anionic, cationic or non-ionic surface active agents or surfactants.

PCT publication WO2006046105 (hereinafter referred to as publication number '105) provides a pharmaceutical dosage form comprising oxcarbazepine having median particle size from about 14 µm to 30 µm. As discussed in '525 patent, we have also found that such compositions do not always provide adequate bioavailability of oxcarbazepine.

Different formulations of oxcarbazepine having similar median particle sizes can have differences in the release profile and variations in the resulting plasma level of oxcarbazepine. This is particularly a problem when the drug is in the form of fine particles.

### OBJECTS OF THE INVENTION

The object of the present invention is to provide novel formulations which are effective orally bioavailable dosage forms of oxcarbazepine and processes for preparing the same.

In general terms, the invention aims to provide a therapeutically effective oral formulation of oxcarbazepine. The invention also aims to provide a formulation with good bioavailability. A related aim of the invention is to provide a formulation with a desirably fast dissolution in biological and physiological media. A further aim is to provide a formulation containing convenient, yet effective median particle sizes that can be prepared, handled and formulated without difficulty.

A further aim is to provide a reliable and reproducible process for the preparation of orally bioavailable dosage forms of oxcarbazepine. Thus it is an aim to provide a formulation in which there is efficient dissolution of the drug even when the drug is not necessarily present as very fine particulates.

A further aim of this invention is to provide a tablet with a high loading of oxcarbazepine with the desired particle size range.

Yet a further aim of this invention is to provide a formulation with suitable release profile for treating partial seizures, epilepsy, convulsions and related conditions.

The present invention satisfies some or all of the above aims.

### SUMMARY OF THE INVENTION

The present invention provides a pharmaceutical dosage forms comprising a mixture of a therapeutically effective amount of oxcarbazepine having median particle size ranging from about 15 µm to about 26 µm and one or more hydrophilic polymers, said mixture being formed by subjecting a suspension comprising said oxcarbazepine and a hydrophilic polymer in a solvent, to mixing in a homogenizer, optionally removing the solvent and converting the said mixture into a dosage form.

We have found that the combination of the hydrophilic polymers and the particular oxcarbazepine median particle size of 15-26 µm is important in ensuring good bioavailability in the formulations of the present invention. We have surprisingly found that the formulation offers the same bioavailability and area under curve (AUC) as prior art formulations having smaller median average particle sizes.

In another aspect of the invention, there is provided a process for preparing a pharmaceutical dosage forms comprising a mixture of a therapeutically effective amount of oxcarbazepine having median particle size ranging from about 15 µm to about 26 µm and one or more hydrophilic polymers, the process comprising
- dissolving the hydrophilic polymer in a solvent to get a solution
- suspending oxcarbazepine in the said solution to form a suspension
- subjecting the said suspension to mixing in a homogenizer
- optionally removing the solvent, and
- converting said mixture into a dosage form.

In another aspect of the invention, there is provided a formulation of oxcarbazepine for the treatment of seizures in patients with epilepsy, wherein the formulation comprises a therapeutically effective amount of oxcarbazepine having median particle size ranging from about 15 µm to about 26 µm and one or more hydrophilic polymers.

In another aspect of the invention, there is provided a use of oxcarbazepine in the preparation of a medicament for the treatment of partial seizures in patients with epilepsy, wherein the medicament comprises a therapeutically effective amount of oxcarbazepine having median particle size ranging from about 15 µm to about 26 µm and one or more hydrophilic polymers.

In each case, the target patient group comprises adults and children aged 4-16.

### DESCRIPTION OF THE FIGURES

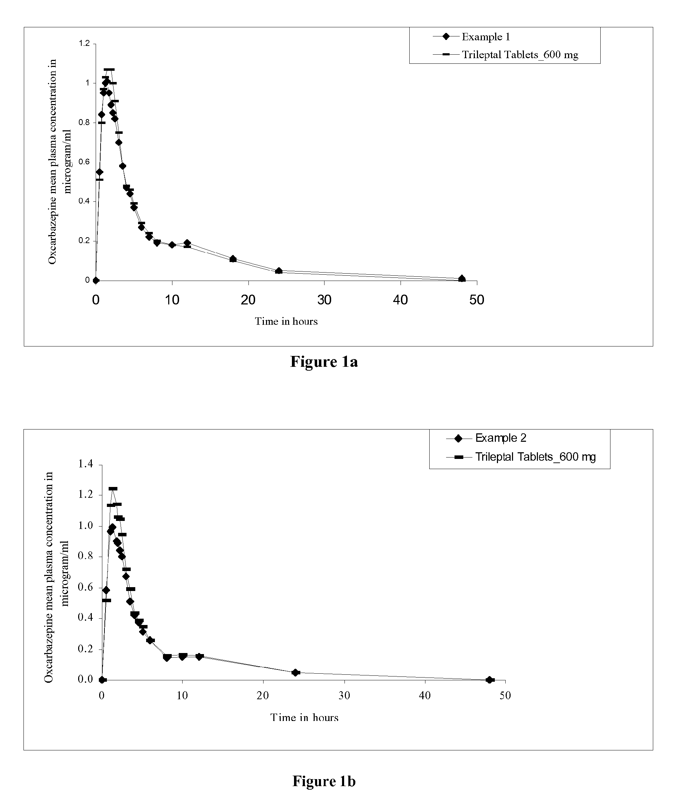

Figure 1 a shows the mean plasma concentration vs. time profile obtained upon administration of an embodiment (described in Example 1) of the present invention having 600 mg oxcarbazepine in comparison to that obtained from an equivalent dose of a dosage form having oxcarbazepine of median particle size of approximately 2 µm to 12 µm commercially available under the trade name of Trilepal^{®}.
Figure 1b shows the mean plasma concentration vs. time profile obtained upon administration of an embodiment (described in Example 2) of the present invention having 600 mg oxcarbazepine in comparison to that obtained from an equivalent dose of a dosage form having oxcarbazepine of median particle size of approximately 2 µm to 12 µm commercially available under the trade name of Trilepal^{®}.

### DETAILED DESCRIPTION OF THE INVENTION

The term therapeutically effective amount of oxcarbazepine used herein means the amount of oxcarbazepine that may be used per unit dosage form ranges from about 100 mg to about 800 mg, preferably about 150 mg to about 700 mg, more preferably 500 to 700 mg and most preferably 600 mg.

According to one embodiment of the present invention, the pharmaceutical dosage form comprises therapeutically effective amount of oxcarbazepine having median particle size (D₅₀) ranging from about 15 µm to about 26 µm. In one embodiment, the median particle size ranges from 15 to not more than 20 µm. In an alternative embodiment, the median particle size ranges from more than 20 to 26 µm. Most preferably, the median particle size ranges from 23 to 26 µm.

The present invention provides pharmaceutical dosage form containing oxcarbazepine having median particle size in the range from about 15 µm to about 26 µm in the form of suitable dosage forms that upon oral administration provides bioavailability comparable to that obtained from an equivalent dose of a dosage form having oxcarbazepine of median particle size of approximately 2 µm to 12 µm available commercially under the trade name of Trilepal^{®} (New Drug Application Number 021014 submitted to the United States Food and Drug Administration, USFDA).

The particle size of oxcarbazepine may be measured using laser light diffraction technique based instrument such as a Malvern Mastersizer Microplus apparatus. Other known methods of measuring particle size may also be used.

In a preferred embodiment of the present invention, the particle size limits for oxcarbazepine were as follows:

| **Particle size** | **Limits** |
|---|---|
| D₁₀* | not more than 10 microns |
| D₅₀* | between 15 to 26 microns |
| D₉₀* | not more than 75 microns |

| | |
|---|---|
| *The term D₁₀ is the size of particle below which 10 % of the sample lies *the term D₅₀ as used herein indicates the size in microns at which 50% of the sample is smaller and 50 % is larger *the term D₉₀ as used herein indicates a size below which 90 % of the sample lies. | |

In an embodiment, D₁₀ is not more than 10 µm, preferably not more than 8 µm. Most preferably, D₁₀ is from 6 to 8 µm. In an embodiment, D₉₀ ranges from 40 to 75 µm, preferably 40 to 50 µm. In an alternative embodiment, D₉₀ ranges from 60 to 70 µm.

In a most preferred embodiment of the present invention, the particle size distribution of oxcarbazepine is such that D₁₀ is not more than 10 microns; D₅₀ is ranging from about 15 µm to about 26 µm and D₉₀ is not more than 50 µm.

According to the present invention, oxcarbazepine is mixed with hydrophilic polymer in a homogenizer. Homogenization may be carried out by a conventional homogenization instruments such as Silverson homogenizer, colloid mill, microfluidizer and the like.

Preferably, the homogenization is carried out at high shear to ensure deaggregation and uniform distribution of oxcarbazepine particles in the liquid medium.

One benefit of the formulations of the invention, is that they have a high loading of the active ingredient. This enables smaller tablets and capsules to be formulated and as such provides a medicament having better patient compliance. This is a significant clinical advantage. Preferably, the loading of oxcarbazepine is greater than or equal to 65% by weight. More preferably, the loading of oxcarbazepine is greater than or equal to 70% by weight.

Another benefit of the formulation of the invention, is that whilst it does not use very fine particulates of oxcarbazepine and have as high a loading, it still provides bioavailability comparable to the formulation of '525 patent having fine particle size. Thus the present invention avoids the need to produce very fine particle sizes in an effective formulation. Another feature is that the invention still provides comparable bioavailability even where the formulations have similar or lower loadings of active than the prior art formulations.

Examples of hydrophilic polymers that are subjected to mixing in a homogenizer may be selected from group comprising of vinyl polymers, cellulose derivatives, polyethylene glycols and mixtures thereof.

Examples of the vinyl polymers that may be used in the present invention include, but are not limited to, polyvinyl pyrrolidone, polyvinyl alcohol and the like. Preferably, polyvinyl pyrrolidone (PVP) with an average molecular weight from about 2000 to about 60,000 may be used in amounts in the range from about 2 % to about 10 % by weight of oxcarbazepine. Higher viscosity grades of polyvinyl pyrrolidone make the process less convenient, but may also be used generally in lower amounts in the range from about 1% to about 5 % by weight of oxcarbazepine.

Examples of the cellulose derivatives that may be used as hydrophilic polymers in the present invention include, but are not limited to, methylcellulose, hydroxypropyl cellulose, hydroxypropyl methylcellulose, hydroxyethyl cellulose and mixtures thereof. Preferably, low viscosity grade cellulose derivatives are used. The low viscosity grade cellulose derivatives that may be used may have a viscosity ranging from about 2 to about 50 mPa, preferably from about 2 to about 15 mPas. These are the viscosity values for 2 % (w/v) aqueous solutions measured at 20°C. Generally, high viscosity grades of cellulose derivatives having viscosity greater than 5 Pas are not recommended and particularly those having viscosity of about 10 Pas may in fact reduce release rate of oxcarbazepine.

Examples of low grade viscosity hydroxy propyl methyl cellulose derivatives that may be used include, but are not limited to, E3 Low viscosity (LV) grade having viscosity of 2.4 to about 3.6, E5 LV having viscosity of 4 to about 6, E6 having viscosity ranging from 5 to 7, E15 having viscosity ranging from 12 to 18; E50 having viscosity ranging from 40 to 60; K3 having viscosity ranging from 2.4 to 3.6.

Examples of Low viscosity grades of hydroxypropyl cellulose that may be used include, but are not limited to, grades that give a viscosity in the range from about 200 mPas to 600 mPas (10 % aqueous solution at 25° C). These are available commercially under the trade name Klucel EF.

Hydroxyethyl cellulose may be used as a hydrophlic polymer according to one embodiment of the present invention. The suitable grades of HEC are those that give viscosity ranging from about 7 mPa to about 150 mPa (5 % aqueous solution at 25°C).

Examples of polyethylene glycol that may be used as hydrophilic polymers that are subjected to mixing in a homogenizer include, but are not limited to, polyethylene glycol having average molecular weights ranging from about 1,000 to 8000, preferably, from about 3000 to about 6000. One preferred embodiment of the present invention uses a mixture of polyethylene glycol 4000 and polyvinyl pyrrolidone as the hydrophilic polymer.

According to one embodiment of the present invention, the hydrophilic polymers and oxcarbazepine are subjected to mixing in a homogenizer in a hydrophilic polymers to oxcarbazepine ratios ranging from about 0.01:1 to about 0.3:1, preferably about 0.02:1 to about 0.08:1, most preferably about 0.06:1.

One of the more preferred embodiments of the present invention uses PVP wherein the ratio between PVP and oxcarbazepine ranges from about 0.04:1 to about 0.06:1. In another more preferred embodiment of the present invention, polyethylene glycol is used as a hydrophilic polymer in combination with PVP wherein the ratio of hydrophilic polymer that is subjected to mixing in a homogenizer to oxcarbazepine is about 0.066:1.

In one of the preferred embodiments of the present invention where the pharmaceutical dosage form is converted into a solid dosage form, the solid dosage form may additionally contain other pharmaceutically acceptable excipients such as wicking agents, disintegrants, binders, lubricants and glidants. Solid dosage forms of the present invention include, but are not limited to, powder, granules, pellets, pills, microspheres, microcapsules, capsules, tablets and the like.

In an embodiment of the present invention, the dosage form is in the form of a tablet. The amount of hydrophilic polymer that is subjected to mixing in a homogenizer may range from about 0.5 % to about 20 % by weight of the tablet. In one of the preferred embodiments of the present invention where the dosage form is a tablet, the amount of hydrophilic polymers that are subjected to mixing in a homogenizer is used in the range of about 2 % to about 10 % by weight of the tablet.

When the solid dosage form of the present invention is in the form of powders, granules, pellets, pills, microcapsules, semisolid and the like, it may be conveniently dispensed in a suitable container preferably, packed in a single unit dose into each container. A suitable single unit dose container that may be used for example, may be a sachet, a capsule and the like.

The solid dosage form of the present invention may also be a semisolid containing mixture of oxcarbazepine and the hydrophilic polymer. The semisolid may be prepared by further mixing or milling or homogenizing the said mixture with pharmaceutically acceptable liquid or semisolid vehicles known in the art. Suitable vehicles that may be used include, polyethylene glycol, propylene glycol, fatty acid triglycerides, emulsifiers and the like.

Examples of wicking agents that may be used in the pharmaceutical dosage form of the present invention include, but are not limited to, colloidal silicon dioxide, kaolin, fumed silicon dioxide, microcrystalline cellulose and the like and mixtures thereof. According to one embodiment of the present invention, the wicking agent may be sifted together with oxcarbazepine. The amount of wicking agent that may be used to sift together with oxcarbazepine may range from about 0.1 % to about 1.0 % by weight of the oxcarbazepine amount, preferably from about 0.3 % to about 0.75 % by weight of the oxcarbazepine amount.

Examples of disintegrants that may be used in the pharmaceutical dosage forms of the present invention may be selected from the group comprising microcrystalline cellulose, starch, e.g., pregelatinized starch and corn starch, croscarmellose sodium, sodium starch glycolate, crospovidone and the like and mixtures thereof. The amount of disintegrants that may be used ranges from about 0.1 % to about 30 % by weight of the total weight of the mixture of oxcarbazepine and all other excipients in the dosage form.

Examples of binders that may be used in the pharmaceutical solid dosage form of the present invention may be selected from the group comprising starch, gelatin, dextrin, maltodextrin, natural and synthetic gums like acacia, carboxymethylcellulose, methylcellulose, hydroxyethyl cellulose, hydroxypropyl cellulose, hydroxypropyl methylcellulose, polyvinylpyrrolidone, polyvinyl alcohol, polyethylene glycol and the like and mixtures thereof. The amount of disintegrants may range from about 0.1 % to about 20 % by weight of the total weight of the mixture of oxcarbazepine and all other excipients in the dosage form.

Examples of the lubricants that may be used in the pharmaceutical solid dosage form of the present invention include, but are not limited to, talc, sodium stearyl fumarate, magnesium stearate, calcium stearate, stearic acid, hydrogenated vegetable oils, polyethylene glycol and the like and mixtures thereof. The amount of disintegrants may range from about 0.01 % to about 5 % by weight of the total weight of the mixture of oxcarbazepine and all other excipients in the dosage form.

Examples of the glidants that may be used in the pharmaceutical solid dosage form of the present invention include, but are not limited to, magnesium stearate, colloidal silicon dioxide, talc and the like. The amount of glidants may range from about 0.01 % to about 1 % by weight of the total weight of the mixture of oxcarbazepine and all other excipients in the dosage form.

The pharmaceutical dosage form of the present invention may be a liquid dosage form. The liquid dosage form of the present invention may contain pharmaceutical excipients such as suspending agents, flocculants or deflocculating agents, flavouring agents, taste masking agents and the like.

According to the process of the present invention, first the hydrophilic polymer(s) are dissolving in a solvent to get a solution. It is possible to use either an aqueous or an organic solvent for dissolving the hydrophilic polymers. Generally, a solvent in which oxcarbazepine is only slightly soluble is used in the present invention. Preferably, the amount of solvent used ranges from about 50 % by weight to about 90 % by weight of oxcarbazepine, more preferably from about 60 % to about 80 % by weight of oxcarbazepine.

Examples of the solvents that may be used include, but are not limited to, purified water, isopropyl alcohol, dichloromethane and the like and mixture thereof. The type of solvent that may be used depends on the nature of hydrophilic polymer that is being used. In one preferred embodiment of the present invention, polyvinyl pyrrolidone is used as the hydrophilic polymer and a mixture of isopropyl alcohol and dichloromethane is used as the solvent.

In one embodiment of the present invention, the amount of hydrophilic polymer that is subjected to mixing in a homogenizer ranges from about 5 % to about 30 % by weight of the solvent, more preferably from about 10 % to about 25 % by weight of the solvent.

According to the process of the present invention, the pharmaceutical dosage form may be prepared by first dissolving the hydrophilic polymer in a solvent. In one of the preferred embodiments, oxcarbazepine is blended with diluent such as Aerosil preferably by sifting the two together through a sieve and the mixture obtained is added with stirring into the solution of hydrophilic polymer in the solvent. The slurry obtained is subjected to mixing in a homogenizer. A slurry that is obtained may be easily flowable to one that is of the paste like consistency that is amenable to mixing in a homogenizer.

In a preferred embodiment of the present invention, a colloid mill is used for mixing. In a more preferred embodiments of the present invention, the clearance between the stator and the rotor of the colloid mill (by CIP Machineries Pvt. Ltd; model: COLLOID HHZ) is adjusted between 5 to 10 units on the attached scale. The speed of the homogenizer may be adjusted to about 5000 rpm. Generally, more than one pass through the homogenizer, for example two passes may be required to get smooth slurry of oxcarbazepine particles in a solution comprising the hydrophilic polymer.

In one embodiment of the present invention, purified water is used as a solvent. The slurry so obtained may itself constitute a suspension dosage form or additional pharmaceutical excipients may be added to obtain a suspension dosage form suitable for oral administration. Alternately, when the solvent includes a non-aqueous solvent, then the solvent may be removed completely or to an acceptable trace levels in further processing, and the dry powder mixture obtained is converted to a suspension using known art and suitable pharmaceutically acceptable excipients.

In one embodiment of the present invention, multiparticulates dosage forms, for example, multiparticulates may be obtained from the slurry. The slurry may be dried to remove the solvent and the dried mass is sieved. The granules may be filled into capsules, sachets or any other suitable unit dose container. In an alternate embodiment, the slurry may be dried until a wet mass is obtained. The wet mass may be granulated by conventional granulation or extrusion-spheronization and the granules may be filled into capsules, sachets or any other suitable unit dose container. Any of the dried multiparticulate mixtures obtained as set forth may be formulated into a suspension dosage form by dispersing them in a suitable pharmaceutically acceptable liquid vehicle. In a preferred embodiment of the present invention, the slurry is converted into tablets. The slurry is mixed with pharmaceutical excipients and granulated to form wet granules. Preferably, the granulation is carried out using heat and vacuum to remove the solvent completely or to pharmaceutically acceptable trace levels.

Preferably no residual solvent is present after drying was completed. For example, in embodiments of the present invention, where dichloromethane is used as a solvent, the residual dichloromethane content is not more than 360 µg per unit dosage form, more preferably it is absent. Similarly, residual isopropyl alcohol content is not more than 3120 µg per unit dosage form, preferably less than 1000 µg per unit dosage form, more preferably less than 800 µg unit dosage form. In preferred embodiments, the total residual solvent (other than water) content is not more than 1000 µg per unit dosage form.

The examples that follow are provided as illustrations and do not limit the scope of the present invention.

### EXAMPLE 1

### Mixing oxcarbazepine and hydrophilic polymer in a homogenizer

A slurry containing oxcarbazepine and hydrophilic polymer in a mixture of isopropyl alcohol and dichloromethane was prepared according to the composition in Table 1 as follows.

**Table 1**

| **Ingredients** | **% by weight** |
|---|---|
| oxcarbazepine | 67.82 |
| colloidal silicon dioxide | 0.34 |
| polyvinyl pyrrolidone (PVP K-30) | 4.07 |
| polyethylene glycol | 0.45 |
| isopropyl alcohol :dichloromethane | 27.31 |

The oxcarbazepine and Aerosil were sifted together through sieve #40 ASTM. The PVP K-30 and polyethylene glycol 4000 were dissolved in 1:1 mixture of isopropyl alcohol and dichloromethane. The oxcarbazepine and Aerosil mixture was added to the solution of hydrophilic polymer under a propeller stirrer. After addition was complete stirring was continued for further 10 minutes. The slurry obtained was passed through colloid mill (by CIP Machineries Pvt. Ltd; model: COLLOID HHZ) and recirculated for 15 minutes at a clearance setting of 7 of the attached scale. The colloid mill was rinsed with minimum amount of solvent mixture.

The particle size distribution of oxcarbazepine was checked after subjecting the slurry of oxcarbazepine and the hydrophilic polymer in a solvent, to mixing in a colloid mill. The particle size was determined by Malvern laser diffraction technique. The particles were dispersed in an aqueous dispersion medium of Tween 20 (0.1 ml/litre). The results are given in Table 2.

**Table 2**

| **Particle size of oxcarbazepine after mixing in a homogenizer** | |
|---|---|
| **% Particles undersized** | **Particle Size of Oxcarbazepine in µm** |
| D (v, 0.1) 10 % particles | 6.93 |
| D (v, 0.5) 50 % particles | 20.56 |
| D (v, 0.91) 90 % particles | 45.97 |

| | |
|---|---|
| v indicates volume; D (v, 0.1) indicates 10 % of particles are less than; D (v, 0.5) indicates 50 % of particles are less than ; D (v, 0.9) indicates 90 % of particles are less than; | |

The slurry containing the homogenized mixture of the present invention was converted into tablet dosage form having a composition given in Table 3 as follows.

**Table 3**

| **ingredients** | **% by weight of the tablet** |
|---|---|
| Oxcarbazepine | 71.38 |
| Colloidal silicon dioxide (Aerosil) | 0.35 |
| **Hydrophilic polymer solution** | |
| Polyvinyl pyrolidone (PVP K-30) | 4.28 |
| Polyethylene glycol 4000 | 0.47 |
| **Granulation** | |
| Microcrystalline cellulose (Avicel PH 101) | 6.42 |
| Croscarmellose Sodium (Ac-Di-Sol) | 1.427 |
| **Extragranular excipients** | |
| Microcrystalline cellulose (Avicel PH 102) | 5.71 |
| crospovidone | 5.71 |
| **Lubrication** | |
| Colloidal silicon dioxide (Aerosil) | 0.83 |
| Sodium stearyl fumarate | 0.95 |
| PVA based film coating | 2.43 |

The slurry was discharged into a jacketed rotary mixer granulator. Steam was circulated through the jacket and vacuum applied. Avicel and crospovidone mixture was added and a wet mass obtained was kneaded. It was passed through sieve # 8 ASTM and dried in a fluid bed at 55°C until the loss of drying was not more than 1.2 % by w/w. This amount is attributable mainly to the trace water that is usually present in isopropyl alcohol.

The dried mass was passed through sieve # 20 ASTM and mixed with lubricants. Granules were compressed into tablets. The tablets were further coated with aqueous polyvinyl alcohol suspension till 2.5 % weight gain is achieved. The residual dichloromethane content was less than 30 µg per tablet and the residual isopropyl alcohol content was less than 800 µg per tablet.

### EXAMPLE 2

### Mixing oxcarbazepine and hydrophilic polymer in a homogenizer

A slurry containing oxcarbazepine (median particle size D₅₀=24.91 µm, D₁₀ =6.59 µm and D₉₀ =63.31 µm) and hydrophilic polymer in a mixture of isopropyl alcohol and dichloromethane was prepared according to the composition in Table 4 as follows.

**Table 4**

| **Ingredients** | **% by weight** |
|---|---|
| oxcarbazepine | 68.13 |
| colloidal silicon dioxide | 0.34 |
| polyvinyl pyrrolidone (PVP K-30) | 4.08 |
| isopropyl alcohol :dichloromethane | 27.43 |

Oxcarbazepine and Aerosil were sifted together. PVP K-30 was dissolved in a 1:1 mixture of isopropyl alcohol and dichloromethane. Oxcarbazepine and Aerosil were added to the PVP-K-30 solution in the solvent under a propeller stirrer. After addition was complete stirring was continued for further 10 minutes. The slurry of oxcarbazepine was passed through colloid mill ((by CIP Machineries Pvt. Ltd; model: COLLOID HHZ) and recirculated for 15 minutes at the clearance setting of 5 of the attached scale. The mill was rinsed with minimum amount of solvent mixture. Rinsing was added to the slurry. The slurry was sprayed in trays and placed in tray dryer set at 35°C.

The slurry containing the homogenized mixture of the present invention was converted into tablet dosage form having a composition given in Table 5 as follows.

**Table 5**

| **Ingredients** | **% by weight of tablet** |
|---|---|
| Oxcarbazepine | 71.9 |
| Aerosil | 0.35 |
| **Hyderophilic polymer solution** | |
| Polyvinyl pyrolidone (PVP K-30) | 4.31 |
| **granulation** | |
| Microcrystalline cellulose (Avicel PH 101) | 6.47 |
| Croscarmellose Sodium (Ac-Di-Sol) | 1.43 |
| **Extra granular excipients** | |
| Microcrystalline cellulose (Avicel PH 102) | 6.23 |
| Croscarmellose sodium | 4.79 |
| **Lubrication** | |
| Colloidal silicon dioxide (Aerosil) | 0.59 |
| Magnesium stearate | 0.95 |
| PVA based film coating | 2.91 |

The dried slurry was removed from oven and mixture of Avicel and croscarmellose was added to it and the mass was kneaded, sieved through 8 # and dried at 55°C. The dried mass was passed through 20 # sieve and lubricated. The granules were compressed into tablets and were coated film coated.

### EXAMPLE 3

The bioavailability of the oral pharmaceutical dosage form (tablet) of oxcarbazepine of the present invention and that of oxcarbazepine tablets containing equivalent dose of oxcarbazepine having a median particle size of approximately 2 µm to about 12 µm, commercially available under the trade name of Trileptal^{®} tablets (600mg) were studied. A single-dose, open label, randomized, comparative and two-way crossover study, with a seven-day washout period, was undertaken for the same.

Healthy male volunteers (n=number of volunteers, n=64 for Example 1 and n=40 for Example 2) were enrolled for two-way crossover study. The subjects were fasted overnight before dosing and for 4 hours thereafter. Drinking water was prohibited 2 hours before dosing and 2 hours thereafter, but was allowed at all other times. Standard meals were provided at 4, 6 and 8 hours after dosing and at appropriate times thereafter. Meal plans were identical for both the periods.

Subjects received a single tablet of oxcarbazepine prepared according to Example 1 and Example 2 respectively (600 mg) with 240 ml of drinking water at ambient temperature as the test medication, and a single oral dose of Trileptal^{®} (600 mg oxcarbazepine) also with 240 ml of drinking water at ambient temperature as the reference medication.

The results of the bioavailability study are given in Figure 1a and 1b. The mean plasma concentrations of oxcarbazepine obtained after administration of the pharmaceutical dosage forms containing oxcarbazepine were plotted against time (post dose) in hour.

Figure 1a and Figure 1b show that the plasma levels obtained by pharmaceutical tablets prepared according to Example 1 and Example 2 respectively, are comparable to the oxcarbazepine tablets having a median particle size of approximately 2 µm to about 12 µm, commercially available under the trade name of Trileptal^{®}. The Area under the plasma concentration vs. time profile curve (AUC) serves as a measure of extent of absorption or in other terms 'bioavailability'. It was found that the AUC or bioavailability of oxcarbazepine from dosage forms of Example 1 and example 2 was 105.03 % and 88.39 % respectively as compared to that oxcarbazepine tablets having a median particle size approximately 2 µm to about 12 µm, commercially available under the trade name Trileptal^{®}. This was surprising in view of United States Patent No. 7, 037,525, which taught that a median particle size of approximately 2 µm to about 12 µm was essential requirement for rendering oxcarbazepine bioavailable upon oral administration.

## Claims

1. A pharmaceutical dosage form comprising a mixture of a therapeutically effective amount of oxcarbazepine having median particle size ranging from about 15 µm to about 26 µm and one or more hydrophilic polymers, said mixture being formed by subjecting a suspension comprising said oxcarbazepine and a hydrophilic polymer in a solvent, to mixing in a homogenizer, optionally removing the solvent and converting the said mixture into a dosage form.

2. A pharmaceutical dosage form as claimed in claim 1 wherein 90 % of the oxcarbazepine particles are less than 50 µm.

3. A pharmaceutical dosage form of claim 1 or claim 2 wherein the dosage form is in the form of a multiparticulate mixture filled into capsules.

4. A pharmaceutical dosage form of claim 1 or claim 2 wherein the dosage form is in the form of a tablet.

5. A pharmaceutical dosage form of claim 1 or claim 2 wherein the dosage form is in the form of a suspension.

6. A pharmaceutical dosage form as claimed in any preceding claim wherein the hydrophilic polymer is selected from the group consisting of polyvinyl pyrrolidone, polyvinyl alcohol, methylcellulose, hydroxypropyl cellulose, hydroxypropyl methyl cellulose, hydroxyethyl cellulose, polyethylene glycol and mixtures thereof.

7. A pharmaceutical dosage form as claimed in claim 6 wherein the hydrophilic polymer is polyethylene glycol having molecular weight ranging from about 3000 to about 6000.

8. A pharmaceutical dosage form as claimed in any preceding claim wherein the ratio of hydrophilic polymer to oxcarbazepine is in the range from about 0.02: 1 to about 0.08:1.

9. A pharmaceutical dosage form as claimed in any preceding claim wherein the hydrophilic polymer is a selected from the group consisting of polyvinyl pyrrolidone, polyethylene glycol and mixtures thereof; and solvent is a mixture of isopropyl alcohol and dichloromethane.

10. A process for preparing a pharmaceutical dosage form comprising a mixture of a therapeutically effective amount of oxcarbazepine having median particle size ranging from about 15 µm to about 26 µm and one or more hydrophilic polymers, the process comprising the steps:
(a) dissolving the hydrophilic polymer in a solvent to obtain a solution
(b) suspending oxcarbazepine in the said solution to form a suspension
(c) subjecting the said suspension to mixing in a homogenizer
(d) optionally removing the solvent, and
(e) converting said mixture into a dosage form.

11. A process as claimed in claim 10 wherein the dosage form is a suspension dosage form.

12. A process as claimed in claim 10 wherein the dosage form is a solid dosage form.

13. A process as claimed in any of claims 10 to 12 wherein the hydrophilic polymer is selected from a group consisting of polyvinyl pyrrolidone, polyethylene glycol and mixtures thereof and the solvent is a mixture of isopropyl alcohol and dichloromethane.

14. A process as claimed in any of claims 10 to 13 wherein the ratio of hydrophilic polymer that is subjected to mixing in a homogenizer to oxcarbazepine ranges from about 0.02:1 to about 0.08:1.

15. A process as claimed in any of claims 10 to 14 wherein the amount of hydrophilic polymer that is subjected to mixing in a homogenizer ranges from about 10 % to about 25 % by weight of the solvent.

16. A process as claimed in claim 12 wherein the total residual solvent (other than water) content is not more than 1000 microgram per unit dosage form.

17. A process as claimed in claim 12 wherein the residual dichloromethane content is not more than 360 microgram per unit dosage form and the residual isopropyl alcohol content is not more than 1000 microgram per unit dosage form.

18. A formulation of oxcarbazepine for the treatment of seizures in patients with epilepsy, wherein the formulation comprises a therapeutically effective amount of oxcarbazepine having median particle size ranging from about 15 µm to about 26 µm and one or more hydrophilic polymers.

19. A use of oxcarbazepine in the preparation of a medicament for the treatment of partial seizures in patients with epilepsy, wherein the medicaments comprises a therapeutically effective amount of oxcarbazepine having median particle size ranging from about 15 µm to about 26 µm and one or more hydrophilic polymers.
